# EUROPEAN PATENT APPLICATION

(11) **EP 2 333 102 A1**
(43) Date of publication of application: **15.06.2011**
(21) Application number: 09179046.9
(22) Date of filing: 14.12.2009
(51) Int. Cl.: C12Q 1/68

(54) **Method for identifying RNA ligands**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner

(57) **Abstract**

The present invention combines a Qß replicase system with an emulsion or micelle based replication technique and SELEX. The invention relates to a method for isolating an RNA ligand from a candidate mixture of RNA molecules, (i) each comprising a segment of least eight contiguous randomized nucleotides, being nucleotides of randomized type, (ii) said RNA ligand being capable of binding a given target, the method comprising the steps of, (iii) contacting the candidate mixture with one or more targets, (iv) partitioning the unbound RNA molecules from the bound RNA ligands, (v) amplifying the RNA ligands with an RNA dependant RNA polymerase in an amplification reaction fluid and (vi) repeating steps (i) to (v) at least 2 times.The present invention also relates to a kit for isolating an RNA ligand from a candidate mixture of RNA molecules comprising a substance for generating micelles and an RNA dependant RNA replicating enzyme.

SEQUENCE LISTING

<110> Max-Planck Gesellschaft zur Förderung der Wissenschaften
e.V.

<120> METHOD FOR IDENTIFYING RNA LIGANDS

<130> R3064 EP BLN

<160> 26

<170> PatentIn version 3.3

<210> 1
<211> 17
<212> DNA
<213> Artificial Sequence

<220>
<223> primer

<400> 1
ggggttccaa ccggaag 17

<210> 2
<211> 42
<212> DNA
<213> Artificial Sequence

<220>
<223> primer

<400> 2
ccggtttcgg acctccagtg cgtgttaccg cactgttagc cc 42

<210> 3
<211> 153
<212> DNA
<213> Artificial Sequence

<220>
<223> ligand

<220>
<221> misc_feature
<222> (54)..(93)
<223> n is a, c, g, or t

<400> 3
taatacgact cactataggg gttccaaccg gaagttgaca ccgtacctgc tctnnnnnnn 60

nnnnnnnnnn nnnnnnnnnn nnnnnnnnnn nnnaagcacg ccagggacta tccggtttcg 120

gacctccagt gcgtgttacc gcactgttag ccc 153

<210> 4
<211> 136
<212> RNA
<213> Artificial Sequence

<220>
<223> ligand

<220>
<221>  misc_feature
<222>  (37)..(76)
<223>  n is a, c, g, or u

<400>  4
gggguuccaa ccggaaguug acaccguacc ugcucunnnn nnnnnnnnnn nnnnnnnnnn      60

nnnnnnnnnn nnnnnnaagc acgccaggga cuauccgguu ucggaccucc agugcguguu     120

accgcacugu uagccc                                                     136


<210>  5
<211>  53
<212>  DNA
<213>  Artificial Sequence

<220>
<223>  primer

<400>  5
taatacgact cactataggg gttccaaccg gaagttgaca ccgtacctgc tct            53


<210>  6
<211>  60
<212>  DNA
<213>  Artificial Sequence

<220>
<223>  primer

<400>  6
gggctaacag tgcggtaaca cgcactggag gtccgaaacc ggatagtccc tggcgtgctt      60


<210>  7
<211>  19
<212>  DNA
<213>  Artificial Sequence

<220>
<223>  primer

<400>  7
gggctaacag tgcggtaac                                                   19


<210>  8
<211>  39
<212>  DNA
<213>  Artificial Sequence

<220>
<223>  ligand

<400>  8
agccttagag cctgtggtgt cccgtttagc ctggagcgc                             39


<210>  9
<211>  40
<212>  DNA
<213>  Artificial Sequence

<220>
<223>  ligand

<400>  9
atctaatagg gcatccgtac gttgttcttg acgatgcttc                              40

<210>  10
<211>  38
<212>  DNA
<213>  Artificial Sequence

<220>
<223>  ligand

<400>  10
agctgtctgc aagagcacaa cggggatggc atgtttgt                                38

<210>  11
<211>  40
<212>  DNA
<213>  Artificial Sequence

<220>
<223>  ligand

<400>  11
ggtacatgtc tctactcacg ttagtcatct agctgccata                              40

<210>  12
<211>  40
<212>  DNA
<213>  Artificial Sequence

<220>
<223>  ligand

<400>  12
acagcatgtg cggtatctcc acacccagtc cgtgctttag                              40

<210>  13
<211>  40
<212>  DNA
<213>  Artificial Sequence

<220>
<223>  ligand

<400>  13
tgcaaaaaga acagagaccg tcaactcttt gtcgtgctag                              40

<210>  14
<211>  40
<212>  DNA
<213>  Artificial Sequence

<220>
<223>  ligand

<400>  14
atgagcgcga taaatagtgt cactctcagc ccgactgcct                              40

<210>  15
<211>  39
<212>  DNA

<213>  Artificial Sequence

<220>
<223>  ligand

<400>  15
tgcaaaggag tgatacggtg gttcggtgct ggctgtaat                              39


<210>  16
<211>  40
<212>  DNA
<213>  Artificial Sequence

<220>
<223>  ligand

<400>  16
ggaagacagt aatctaccaa caggcggcag acgtgcaagt                             40


<210>  17
<211>  39
<212>  DNA
<213>  Artificial Sequence

<220>
<223>  ligand

<400>  17
tgcaaaagaa cagagaccgt caactctttg tcgtgctag                              39


<210>  18
<211>  40
<212>  DNA
<213>  Artificial Sequence

<220>
<223>  ligand

<400>  18
gtgacaatca gggctggtag cggggacccg acgtgttgat                             40


<210>  19
<211>  40
<212>  DNA
<213>  Artificial Sequence

<220>
<223>  ligand

<400>  19
gataaagtgc agggctagtg cctcttctgc gcgctaatct                             40


<210>  20
<211>  40
<212>  DNA
<213>  Artificial Sequence

<220>
<223>  ligand

<400>  20
ttgaggagca agggagtgta aaaagaccct cccgcgcgtt                             40

<210> 21
<211> 40
<212> DNA
<213> Artificial Sequence

<220>
<223> ligand

<400> 21
taatgaggag gccgccaaca gccgtcctgc atgctattaa 40


<210> 22
<211> 40
<212> DNA
<213> Artificial Sequence

<220>
<223> ligand

<400> 22
ctgggcagga gggaaatcaa ctggacctgg gtcgctttat 40


<210> 23
<211> 40
<212> DNA
<213> Artificial Sequence

<220>
<223> ligand

<400> 23
cgaagtagta tcgacgagcg gtgtggcttg acgttgctag 40


<210> 24
<211> 40
<212> DNA
<213> Artificial Sequence

<220>
<223> ligand

<400> 24
atgagcgcga taaatagtgt cactctcagc ctgactgcct 40


<210> 25
<211> 15
<212> DNA
<213> Artificial Sequence

<220>
<223> ligand

<400> 25
atgcctatca tgggc 15


<210> 26
<211> 40
<212> DNA
<213> Artificial Sequence

<220>
<223> ligand

<400> 26
gttaatgaag attactgagc aagtgcgtgg gcctgcctat 40

## Description

### FIELD OF THE INVENTION

This invention is in the field of biology, chemistry, molecular biology, more in particular in the field of ligand screening, more in particular in the field of RNA ligand screening as in SELEX, *i.e.* "Systematic Evolution of Ligands by Exponential enrichment".

### BACKGROUND OF THE INVENTION

Selex ("Systematic evolution of ligands by exponential enrichment") also referred to as *in vitro* selection or *in vitro* evolution, is a combinatorial technique in molecular biology in producing oligonucleotides of either single-stranded DNA or RNA that specifically bind to a target ligand or ligands (Ellington, A. D. and Szostak, J. W. (1990) Nature, London, 346, 818-22). The selected sequences are referred to as aptamers. Aptamers are molecules that bind to a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications. Nucleic acid aptamers are a nucleic acid species that have been engineered through repeated rounds and *in vitro* selection or, as outlined above, through SELEX to bind to various molecular targets such as small molecules, proteins, nucleic acids, even cell tissues in organism. Aptamers are useful in biotechnological and therapeutic applications as they offer molecular recognition properties that rival that of the commonly used biomolecule, antibodies. In addition to their discriminate recognition, aptamers offer advantages over antibodies as they can be engineered completely in a test tube, are readily produced by chemical synthesis and possess desirable storage properties and elicit little or no immunogenicity in therapeutic applications. In 1990, two laboratories independently developed the technique of selection: the Gold lab using the term SELEX for their process of selecting RNA ligands against T4 DNA polymerase; and the Szostak lab, coining the term *in vitro selection,* selecting RNA ligands against various organic dyes. The Szostak lab also coined the term aptamer (from the latin, *aptus,* meaning "to fit") for these nucleic acid-based ligands. Two years later, the Szostak lab and Gilead Sciences, independent of one another, used *in vitro selection* schemes to evolve single stranded DNA ligands for organic dyes and human coagulant, thrombin, respectively. Interestingly enough, the notion of selection *in vitro* was actually preceded twenty-plus years prior when Sol Spiegelman used a Qbeta replication system as a way to evolve a self-replicating molecule. In addition, a year before the publishing of *in vitro selection* and SELEX, Gerald Joyce used a system that he termed "directed evolution" to alter the cleavage activity of a ribozyme. Since the discovery of aptamers, many researches have used aptamer selection as a means for application and discovery.

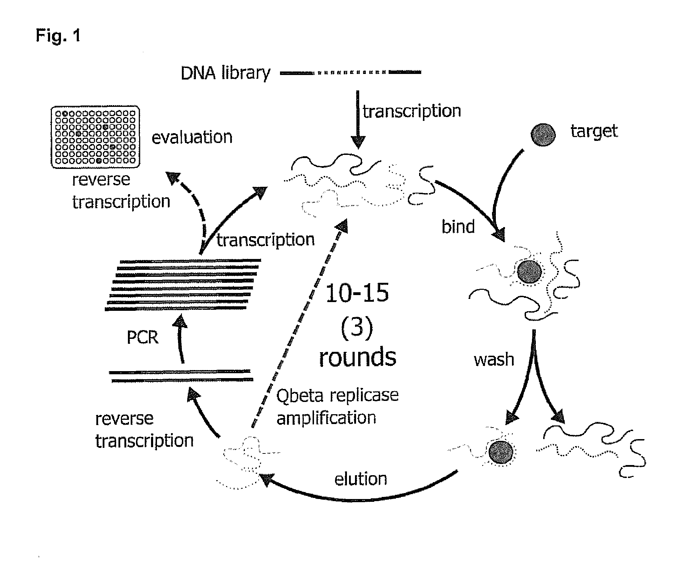

In SELEX the process begins with the synthesis of a very large oligonucleotide library consisting of randomly generated sequences of fixed length flanked by constant 5' and 3' ends that serve as primers; see Fig 1. For a randomly generated region of length n, the number of possible sequences in the library is 4ⁿ. The sequences in the library are exposed to the target ligand - which may be a protein or a small organic compound - and those that do not bind the target are removed, usually by affinity chromatography. The bound sequences are eluted and amplified by polymerase chain reaction (PCR) to prepare for subsequent rounds of selection in which the stringency of the elution conditions is increased to identify the tightest-binding sequences. An advancement of the original method allows an RNA library to omit the constant primer regions, which can be difficult to remove after the selection process because they stabilize secondary structures that are unstable when formed by the random region alone (Jarosch, F., Buchner, K., Klussmann, S. (2006). In vitro selection using a dual RNA library that allows primerless selection. Nucleic Acids Res 34(12):e86). The presently available SELEX methods and improvements, however, have a number substantial draw backs. The DNA based aptamers SELEX process requires multiple cDNA generation steps whereby in a first step the initial library is turned into RNA bound to the desired target and retransferred into DNA for amplification purposes. Then again RNA regeneration is performed. This process per se is not just time consuming but involves multiple opportunities for errors and failure. At the same time the DNA based SELEX process including cloning and sequencing of the clones may take as long as ten weeks. RNA based SELEX processes, however, so far had the draw back that replication of RNA molecules is difficult and prone to preferential amplification of certain RNA molecules which not necessarily are those that have the desired target binding property. In a previously known method contaminations or parasitic "monsters" were created that tended to supersede sequences of interest. The present inventors have now found ways of substantially improving on the SELEX process. The advantages of the invention lie in the rapid turnaround times per selection round as well as the reduced number of selection rounds as well as the dramatically improved efficiency, wherein parasitic "monsters" are prohibited from establishing during the reaction, all in all leading to a dramatically improved SELEX process.

### SUMMARY OF THE INVENTION

The present invention combines a Qß replicase system with an emulsion or micelle based replication technique and SELEX. The invention relates to a method for isolating an RNA ligand from a candidate mixture of RNA molecules, (i) each comprising a segment of least eight contiguous randomized nucleotides, being nucleotides of randomized type, (ii) said RNA ligand being capable of binding a given target, the method comprising the steps of, (iii) contacting the candidate mixture with one or more targets, (iv) partitioning the unbound RNA molecules from the bound RNA ligands, (v) amplifying the RNA ligands with an RNA dependant RNA polymerase in an amplification reaction fluid and (vi) repeating steps (i) to (v) at least 2 times.

The present invention also relates to a kit for isolating an RNA ligand from a candidate mixture of RNA molecules comprising a substance for generating micelles and an RNA dependant RNA replicating enzyme.

Herein, an "RNA ligand" means an RNA acid that binds another molecule (target). In a population of RNA molecules, a ligand is one which binds with greater affinity than that of the bulk population. In a candidate mixture there can exist of more than one RNA ligand for a given target. The RNA ligands can differ from one another in their binding affinities for the target molecule.

Herein, a "candidate mixture" is a mixture of RNA molecules of differing sequence, from which to select a desired RNA ligand. The source of a candidate mixture is chemical synthesis or enzymatic synthesis of RNA molecules.

Herein, a "target" molecule means any compound of interest for which an RNA ligand is desired. A target molecule can be a protein, peptide, carbohydrate, polysaccharide, glycoprotein, hormone, receptor, cell surface, antigen, antibody, toxin, virus, substrate, metabolite, transition state analogue, cofactor, inhibitor, drug, dye, nutrient, growth factor, etc., without limitation.

Herein, partitioning means any process whereby the increased affinity RNA ligands are separated from the remainder of the candidate mixture. Thus, it includes a process whereby RNA ligands bound to target molecules, termed ligand-target pairs herein, can be separated from RNA molecules not bound to target molecules. Partitioning can be accomplished by various methods known in the art. RNA ligand protein pairs can be bound to nitrocellulose filters while unbound RNA moelcules are not. Columns which specifically retain RNA ligand-target pairs (or specifically retain bound ligand complexed to an attached target) can be used for partitioning. Liquid-liquid partition can also be used as well as filtration gel retardation, and density gradient centrifugation. The choice of partitioning method will depend on properties of the target and of the RNA ligand-target pairs and can be made according to principles and properties known to those of ordinary skill in the art.

Amplifying means any process or combination of process steps that increases the amount or number of copies of a molecule or class of molecules making use of an RNA dependant RNA replicating enzyme. Amplifying RNA molecules in the disclosed examples was carried out NOT by a sequence of three reactions: making cDNA copies of selected RNAs, using polymerase chain reaction to increase the copy number of each cDNA, and transcribing the cDNA copier to obtain RNA molecules having the same sequences as the selected RNAs but in contrast to the prior art by replicating the RNA directly.

The terms "microcapsule or micelle or emulsion" are all used and mean the same. In essence, however, a microcapsule is an artificial compartment whose delimiting borders restrict the exchange of the components of the replication reaction with the RNA replicase. Ideally one or only a few replication templates are in each micelle.

Herein, a "high replicating flank sequence" is an RNA sequence in an RNA molecule which will be replicated in an exponential manner by an RNA dependant RNA polymerase wherein ideally the polymerase does not dissociate from its template.

### FIGURE CAPTIONS

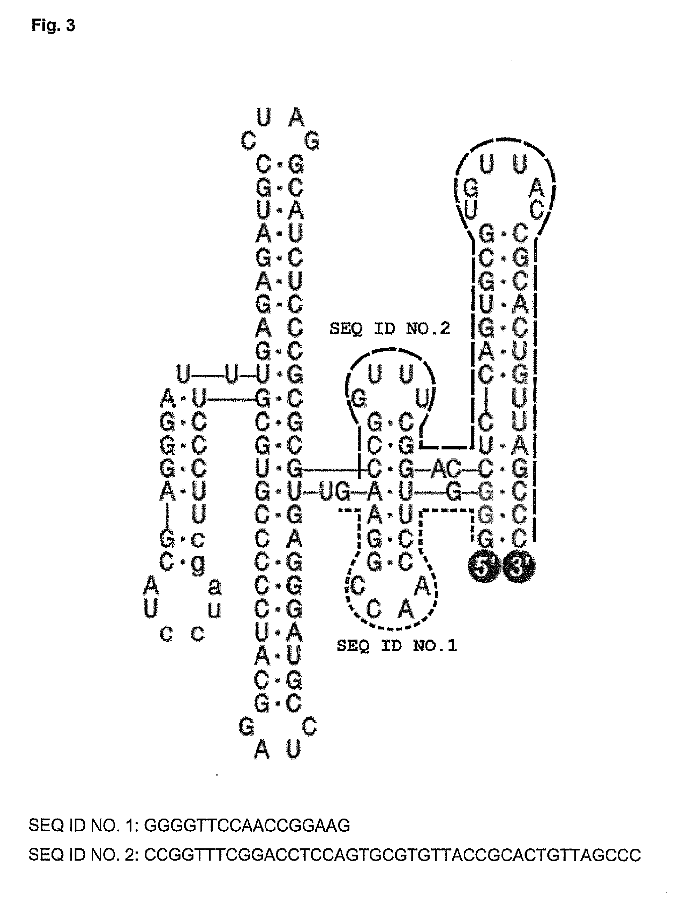

**Fig. 1**
   Selection scheme for RNA SELEX. The repetitive part of the protocol is represented in the circle of arrows. The new protocol using Qbeta replicase mediated amplification is indicated by the dashed arrow bypassing conventional reverse transcription, PCR, and transcription steps.
**Fig. 2**
   Sequence traces derived from batch sequencing of PCR fragments by the sanger protocol. A) represents the original library before the selection experiment, clearly showing the grafted variable sequence. B) represents two serial transfers of the library without any emulsion during amplification resulting in a clearly defined single sequence. C) represents the third selection round on Streptavidin with amplification in emulsion with bias towards certain bases, but remaining variability in the grafted sequence.
**Fig. 3**
   The inventors have taken the high replicating flank sequences from the molecule depicted in Fig. 3 and transferred them as fixed sequences to the 3' and 5' end of each RNA molecule in the library. High replicating flank sequences are indicated by dashed lines.

### DETAILED DESCRIPTION OF THE INVENTION

The Qß replicase system was initially described in 1388. The Qß replicase system is based on the incorporation of a single-stranded oligonucleotide probe into an RNA molecule that can be exponentially amplified after target hybridization by the enzyme Qß replicase. The present inventors have astonishingly found that very good selection results may be achieved by combining the Qß replicase system with an emulsion or micelle based replication technique. Hence, the invention relates to a method for isolating an RNA ligand from a candidate mixture of RNA molecules, (i) each comprising a segment of least eight contiguous randomized, (ii) nucleotides, being nucleotides of randomized type, (iii) said RNA ligand being capable of binding a given target, the method comprising the steps of, (iv) contacting the candidate mixture with one or more targets, (v) partitioning the unbound RNA molecules from the bound RNA ligands, (vi) amplifying the RNA ligands with an RNA dependant RNA polymerase in an amplification reaction fluid and (vii) repeating steps (i) to (vi) at least 2 times.

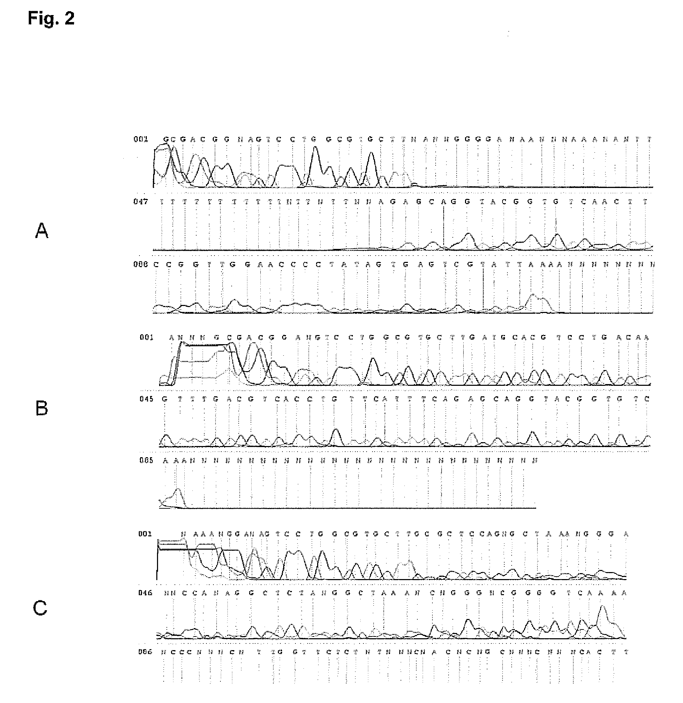

The present method is less error prone, much faster then standard SELEX and only after very few rounds generates RNA ligands capable of binding a given target; see Fig. 2.

RNA motifs is a term generally used to describe the secondary or tertiary structure of RNA molecules. The primary sequence of an RNA is a specific string of nucleotides (A, C, G or U) in one dimension. The primary sequence does not give information on first impression as to the three dimensional configuration of the RNA, although it is the primary sequence that dictates the three dimensional configuration. In certain cases, the RNA ligand solution obtained after performing the method of the invention on a given target may best be represented as a primary sequence. Although conformational information pertaining to such an RNA ligand solution is not always ascertainable based on the results obtained by the method, the representation of an RNA ligand solution as a primary sequence shall not be interpreted as disclaiming the existence of an integral tertiary structure. The secondary structure of an RNA motif is represented by contact in two dimensions between specific nucleotides. The most easily recognized secondary structure motifs include the Watson/Crick basepairs A U and C:G. Non-Watson/Crick basepairs, often of lower stability, have been recognized, and include the pairs G:U, AC, G:A, and U:U.(Base pairs are shown once; in RNA molecules the base pair XY by convention represents a sequence in which X is 5' to Y, whereas the base pair Y:X is also allowed.) The conventional nomenclature for the secondary structures includes hairpin loops, asymmetric bulged hairpin loops, symmetric hairpin loops, and pseudoknots. When nucleotides that are distant in the primary sequence and not thought to interact through Watson/Crick and non-Watson/Crick base pairs are in fact interacting, these interactions (which are often depicted in two dimensions) are also part of the secondary structure. The three dimensional structure of an RNA motif is merely the description, in space, of the atoms of the RNA motif. Double-stranded RNA, fully base paired through Watson/Crick pairing, has a regular structure in three dimensions, although the exact positions of all the atoms of the helical backbone could depend on the exact sequence of bases in the RNA. A vast literature is concerned with secondary structures of RNA motifs, and those secondary structures containing Watson/Crick base pairs are thought often to form A-form double stranded helices. From A-form helices one can extend toward the other motifs in three dimensions. Non-Watson/Crick base pairs, hairpin loops, bulges, and pseudoknots are structures built within and upon helices. Primary sequences of RNAs limit the possible three dimensional structures, as do the fixed secondary structures. The three dimensional structures of an RNA are limited by the specified contacts between atoms in two dimensions, and are then further limited by energy minimizations, the capacity of a molecule to rotate all freely rotatable bonds such that the resultant molecule is more stable than other conformers having the same primary and secondary sequence and structure.

Ligand solutions are defined as the three dimensional structure, held in common or as a family, that defines the conserved components identified through the present invention. Thus, RNA ligand solutions are meant to represent a potentially large collection of appropriate sequence/structures, each identified by the family description which is inclusive of all exact sequence/structure solutions.

It is further contemplated through this definition that RNA ligand solutions need not include only members with exact numerical equivalency between the various components of an RNA motif. Some RNA ligands may have loops, for example, of five nucleotides while other ligands for the same target may contain fewer or more nucleotides in the equivalent loop and yet be included in the description of the ligand solution.

Although the ligand solution derived by the present invention may include a relatively large number of potential members, the RNA ligand solutions are target-specific and, for the most part, each member of the ligand solution family can be used as an RNA antibody to the target.

The method of the invention can include the initial preparation of a candidate mixture. The individual test RNA nucleic acids contain a randomized region, which can be flanked by sequences conserved in all RNAs in the mixture. The conserved regions are provided to, e.g. at one stage facilitate amplification or selected nucleic acids or as so called "graft" sequences. Thus, the RNA can contain subportions that are randomized, along with subportions which are held constant in all RNA species in the mixture. For example, sequence regions known to bind, or selected for binding, to the target protein can be integrated with randomized regions to achieve improved binding or improved specificity of binding. Sequence variability in the test mixture can also be introduced or augmented by generating mutations in the RNAs in the test mixture during the selection/Qß replication process. In principle, the RNAs employed in the test mixture can be any length as long as they can be amplified. The randomized segment of the RNAs in the candidate mixture can be derived in a number of ways. For example, full or partial sequence randomization can be readily achieved by direct chemical synthesis of the RNA (or portions thereof) or by synthesis of a template from which the RNA can be prepared by use of appropriate enzymes. In those cases in which randomized sequence is employed, it is not necessary (or possible from long randomized segments) that the test mixture contains all possible variant sequences. It will generally be preferred that the candidate mixture contain as large a number of possible sequence variants as is practical for selection, to insure that a maximum number of potential binding sequences are identified. A randomized sequence of 30 ribonucleotides will contain a calculated 1018 different candidate sequences. As a practical matter, it is convenient to sample only about 1015 candidates in a single selection. It is basic to the method than the RNAs of the test mixture are capable of being replicated by an RNA dependant RNA replicase. Thus, it is preferred that any conserved regions employed in the test RNAs do not contain sequences which interfere with RNA replication.

It is, therefore, a preferred embodiment of this invention when utilizing a candidate mixture with an ideally contiguous randomized region, that the randomized region is at least 20 ribonucleotides in length, better even 25 ribonucleotides in length, ideally about 40 ribonucleotides in length. Longer stretches are possible.

An RNA molecule in the candidate mixture may be comprised of fixed sequences flanking the randomized segment that aid in the replication of the selected RNA sequences such as high replicating flank sequence. In one embodiment such a fixed sequence is ideally a high replicating flank sequence. Fig.3 shows the origin two high replicating flank sequences (herein SEQ ID NO. 1 and 2).

Candidate mixtures may also be prepared containing both randomized sequences and fixed sequences wherein the fixed sequences serve a function in addition to the replication process. Such fixed sequences may further stabilise the RNA molecule against the degradation by nucleases. In this stabilised form these RNA molecules may be more readily produced *in vivo. (*Appl Environ Microbiol. 2009 Dec 4., Extracellular production of an RNA aptamer by ribonuclease-less marine bacteria harboring engineered plasmids: a proposal for industrial RNA drug production, Suzuki H, Ando T, Umekage S, Tanaka T, Kikuchi Y*)* Additionally, they can be used as target sequences for specific hydridisation in detection, immobilisation, or reverse transcription experiments.

In one embodiment of the invention, the fixed sequences in a candidate mixture may be selected in order to enhance the percentage of RNAs in the candidate mixture possessing a given RNA motif. For example, the incorporation of the appropriate fixed ribonucleotides will make it possible to increase the percentage of pseudoknots or hairpin loops in a candidate mixture. A candidate mixture that has been prepared including fixed sequences that enhance the percentage of a given RNA structural motif is, therefore, a part of this invention.

It is preferred that the amplification reaction fluid comprises micelles or also called microcapsules.

The formation and the composition of the microcapsules must not abolish the function of the Qß replicase. The appropriate system(s) may vary depending on the precise nature of the requirements in each application of the invention, as will be apparent to the skilled person. A wide variety of microencapsulation procedures are available (see Benita, 1996) and may be used to create the microcapsules used in accordance with the present invention. Indeed, more than 200 microencapsulation methods have been identified in the literature (Finch, 1993). These include membrane enveloped aqueous vesicles such as lipid vesicles (liposomes) (New, 1990) and non-ionic surfactant vesicles (van Hal et al., 1996). These are closed-membranous capsules of single or multiple bilayers of non-covalently assembled molecules, with each bilayer separated from its neighbour by an aqueous compartment. In the case of liposomes the membrane is composed of lipid molecules; these are usually phospholipids but sterols such as cholesterol may also be incorporated into the membranes (New, 1990). A variety of enzyme-catalysed biochemical reactions, including RNA and DNA polymerisation, can be performed within liposomes (Chakrabarti et al., 1994; Oberholzer et al., 1995a; Oberholzer et al., 1995b; Walde et al., 1994; Wick & Luisi, 1996). With a membrane-enveloped vesicle system much of the aqueous phase is outside the vesicles and is therefore non-compartmentalised. This continuous, aqueous phase should be removed or the biological systems in it inhibited or destroyed (for example, by digestion of nucleic acids with DNase or RNase) in order that the reactions are limited to the microcapsules (Luisi et al., 1987). Enzyme-catalysed biochemical reactions have also been demonstrated in microcapsules generated by a variety of other methods. Many enzymes are active in reverse micellar solutions (Bru & Walde, 1991; Bru & Walde, 1993; Creagh et al., 1993; Haber et al., 1993; Kumar et al., 1989; Luisi & B., 1987; Mao & Walde, 1991; Mao et al., 1992; Perez et al., 1992; Walde et ai., 1994; Walde et al., 1993; Walde et al., 1988) such as the AOT-isooctane-water system (Menger & Yamada, 1979). Microcapsules can also be generated by interfacial polymerisation and interfacial complexation (Whateley, 1996). Microcapsules of this sort can have rigid, nonpermeable membranes, or semipermeable membranes. Semipermeable microcapsules bordered by cellulose nitrate membranes, polyamide membranes and lipid-polyamide membranes can all support biochemical reactions, including multienzyme systems (Chang, 1987; Chang, 1992; Lim, 1984). Alginate/polylysine microcapsules (Lim & Sun, 1980), which can be formed under very mild conditions, have also proven to be very biocompatible, providing, for example, an effective method of encapsulating living cells and tissues (Chang, 1992; Sun et al., 1992). Non-membranous microencapsulation systems based on phase partitioning of an aqueous environment in a colloidal system, such as an emulsion, may also be used. Preferably, the microcapsules of the present invention are formed from emulsions; heterogeneous systems of two immiscible liquid phases with one of the phases dispersed in the other as droplets of microscopic or colloidal size (Becher, 1957; Sherman, 1968; Lissant, 1974; Lissant, 1984). Emulsions may be produced from any suitable combination of immiscible liquids. Preferably the emulsion of the present invention has water (containing the biochemical components) as the phase present in the form of finely divided droplets (the disperse, internal or discontinuous phase) and a hydrophobic, immiscible liquid (an 'oil') as the matrix in which these droplets are suspended (the nondisperse, continuous or external phase). Such emulsions are termed 'water-in-oil' (W/O). This has the advantage that the entire aqueous phase containing the biochemical components is compartmentalised in discreet droplets (the internal phase). The external phase, being a hydrophobic oil, generally contains none of the biochemical components and hence is inert. The emulsion may be stabilised by addition of one or more surface-active agents (surfactants). These surfactants are termed emulsifying agents and act at the water/oil interface to prevent (or at least delay) separation of the phases. Many oils and many emulsifiers can be used for the generation of water-in-oil emulsions; a recent compilation listed over 16,000 surfactants, many of which are used as emulsifying agents (Ash and Ash, 1993). Suitable oils include light white mineral oil and non-ionic surfactants (Schick, 1966) such as sorbitan monooleate (Span™80; ICI) and polyoxyethylenesorbitan monooleate (Tween ™80; ICI). The use of anionic surfactants may also be beneficial. Suitable surfactants include sodium cholate and sodium taurocholate. Particularly preferred is sodium deoxycholate, preferably at a concentration of 0.5% w/v, or below.

Creation of an emulsion generally requires the application of mechanical energy to force the phases together. There are a variety of ways of doing this which utilise a variety of mechanical devices, including stirrers (such as magnetic stir-bars, propeller and turbine stirrers, paddle devices and whisks), homogenisers (including rotor-stator homogenisers, high-pressure valve homogenisers and jet homogenisers), colloid mills, ultrasound and 'membrane emulsification' devices (Becher, 1957; Dickinson, 1994). Aqueous microdroplets formed in water-in-oil emulsions are generally stable with little if any exchange of genetic elements or gene products between microcapsules. Additionally, we have demonstrated that several biochemical reactions proceed in emulsion microdroplets. Moreover, complicated biochemical processes, notably gene transcription and translation are also active in emulsion microdroplets. The technology exists to create emulsions with volumes all the way up to industrial scales of thousands of litres (Becher, 1957; Sherman, 1968; Lissant, 1974; Lissant, 1984). The preferred microdroplet size will vary depending upon the precise requirements of any individual selection process that is to be performed according to the present invention. In all cases, there will be an optimal balance between RNA library size, the required enrichment and the required concentration of components in the individual microdroplets to achieve efficient Qß replication. The processes of replication ideally occurs within each individual microdroplet provided by the present invention. Because of the requirement for only a limited number of RNA molecules to be present in each microcapsule, this therefore sets a practical upper limit on the possible microdroplet size. Preferably, the mean volume of the microdroplet is less that 5.2 x 10-16 m³, (corresponding to a spherical microdroplet of diameter less than 10µm), more preferably less than 6.5 x 10-17 m³ (5µm), more preferably about 4.2 x 10-18 m³ (2µm) and ideally about 9 x 10-18 m³ 20 (2.6µm). Ideally the diameter is between 2 µm and 10 µm, better even between 3 µm and 9 µm and best between 4 µm and 8 µm.

The microdroplet size must be sufficiently large to accommodate all of the required components of the Qß replication reaction that are needed to occur within the microcapsule.

According to a further aspect of the present invention, there is provided a method for compartmentalising said RNA ligand being capable of binding a given target after partitioning the unbound RNA molecules from the bound RNA ligands and prior to amplifying the RNA ligands with an RNA dependant RNA polymerase in an amplification reaction fluid, comprising the steps of (i) forming an aqueous solution comprising the one or more RNA ligands and the Qß replicase (ii) microencapsulating the solution so as to form a discrete microcapsule comprising the one or more RNA ligands and the Qß replicase and (iii) exposing the microcapsule to conditions suitable for RNA replication.

It is further preferred that the micelles may comprise one or more of the following substances, an oil, a mineral oil, an ionic surfactant, a non-ionic surfactant, an organic solvent.

Surfactants may be selected from the group of, an anionic surfactant based on sulfate, sulfonate or carboxylate anions, perfluorooctanoate (PFOA or PFO), perfluorooctanesulfonate (PFOS), ammonium lauryl sulfate, other alkyl sulfate salts, sodium laureth sulphate, alkyl benzene sulfonate, soaps, or fatty acid salts, cationic surfactants (based on quaternary ammonium cations), cetyl trimethylammonium bromide (CTAB) a.k.a. hexadecyl trimethyl ammonium bromide, alkyltrimethylammonium salts, cetylpyridinium chloride (CPC), polyethoxylated tallow amine (POEA), benzalkonium chloride (BAC), benzethonium chloride (BZT), zwitterionic (amphoteric), dodecyl betaine, cocamidopropyl betaine, coco ampho glycinate, nonionic surfactants, alkyl poly(ethylene oxide), alkylphenol poly(ethylene oxide), copolymers of poly(ethylene oxide) and poly(propylene oxide), alkyl polyglucosides, octyl glucoside, decyl maltoside, fatty alcohols, cetyl alcohol, oleyl alcohol, cocamide MEA, cocamide DEA, polysorbates, Tween 20, Tween 80 and dodecyl dimethylamine oxide.

In a preferred embodiment the RNA dependant RNA polymerase in the amplification reaction fluid is Qß replicase.

Other possible RNA dependant RNA polymerases are those derived from related *Leviviridae* like MS2, and other ssRNA viruses or those from dsRNA viruses such as *Totiviridae.* A mixture of such polymerases may be used to enhance amplification for a greater range of sequences.

The volume of the replication is preferably between 10µl and 1ml. Ideally, about between 0.2 and '10 units of Qß replicase are in the reaction (10µl bis 1 ml). We have found that best results are achieved when incubating at a temperature of between 21°C and 37°C for between 1 and 4 hours.

It is preferred that the RNA molecules comprise a fixed sequence which enhances replication by the Qß replicase, *i.e.* a high replicating flank sequence.

Ideally the high replicating flank sequences are selected from the pairs of SEQ ID NO. 1 and 2.

Preferably the one or more of the RNA ligands is sequenced directly without prior cloning. An aliquot of the replication reaction is sequenced directly; see Fig. 2. One possible method is in *vitro* clonal amplification. Molecular detection methods are not sensitive enough for single molecule sequencing, so most approaches use an in vitro cloning step to amplify individual DNA molecules. Emulsion PCR isolates individual DNA molecules along with primer-coated beads in aqueous droplets within an oil phase. Polymerase chain reaction (PCR) then coats each bead with clonal copies of the DNA molecule followed by immobilization for later sequencing. Emulsion PCR is used in the methods by Marguilis *et al.* (commercialized by 454 Life Sciences), Shendure and Porreca et al. (also known as "Polony sequencing") and SOLiD sequencing, (developed by Agencourt, now Applied Biosystems)(Margulies M, Egholm M, Altman WE, et al (September 2005). "Genome sequencing in microfabricated high-density picolitre reactors". Nature 437 (7057): 376-80: Nature; Shendure, J. (2005). "Accurate Multiplex Polony Sequencing of an Evolved Bacterial Genome". Science 309). Another method for in *vitro* clonal amplification is bridge PCR, where fragments are amplified upon primers attached to a solid surface, used in the Illumina Genome Analyzer. The single-molecule method developed by Stephen Quake's laboratory (later commercialized by Helicos) skips this amplification step, directly fixing DNA molecules to a surface (Braslavsky I, Hebert B, Kartalov E, Quake SR (April 2003). "Sequence information can be obtained from single DNA molecules". Proc. Natl. Acad. Sci. U.S.A. 100 (7))

In parallelized sequencing, DNA molecules are physically bound to a surface, and sequenced in parallel. Sequencing by synthesis, like dye-termination electrophoretic sequencing, uses a DNA polymerase to determine the base sequence. Reversible terminator methods (used by lllumina and Helicos) use reversible versions of dye-terminators, adding one nucleotide at a time, detect fluorescence at each position in real time, by repeated removal of the blocking group to allow polymerization of another nucleotide. Pyrosequencing (used by 454) also uses DNA polymerization, adding one nucleotide species at a time and detecting and quantifying the number of nucleotides added to a given location through the light emitted by the release of attached pyrophosphates (M. Ronaghi, S. Karamohamed, B. Pettersson, M. Uhlen, and P. Nyren (1996). "Real-time DNA sequencing using detection of pyrophosphate release". Analytical Biocherrmistry 242: 84-9).

Sequencing by ligation uses a DNA ligase to determine the target sequence. Used in the polony method and in the SOLiD technology, it uses a pool of all possible oligonucleotides of a fixed length, labeled according to the sequenced position. Oligonucleotides are annealed and ligated; the preferential ligation by DNA ligase for matching sequences results in a signal informative of the nucleotide at that position.

In microfluidic Sanger sequencing the entire thermocycling amplification of DNA fragments as well as their separation by electrophoresis is done on a single glass wafer (approximately 10 cm in diameter) thus reducing the reagent usage as well as cost.

In one embodiment of the invention an aliquot of the replication is taken after each round of selection and sequenced directly. In another embodiment this is done after 2 or more rounds. It is possible to determine a target RNA ligand sequence also in parts, *i.e.* only just a consensus sequence or binding motif.

An embodiment of the present invention, which is particularly useful for the identification or isolation of RNAs which bind to a particular functional or active site in a protein, or other target molecule, employs an RNA molecule known, or selected, for binding to a desired site within the target protein to direct the selection/amplification process to a subset of RNA ligands that bind at or near the desired site within the target molecule. In a simple example, an RNA sequence known to bind to a desired site in a target molecule is incorporated near the randomized region of all RNAs being tested for binding. The present method is then used to select those variants, all of which will contain the known binding sequence, which bind most strongly to the target molecule. A longer binding sequence, which is anticipated to either bind more strongly to the target molecule or more specifically to the target can thus be selected. This also makes the creation bi-functional molecules possible.

The invention also relates to a kit for isolating an RNA ligand from a candidate mixture of RNA molecules comprising a substance for generating micelles and an RNA dependant RNA replicating enzyme.

Preferably the RNA replicating enzyme is Qß replicase.

The kit may additionally comprise the candidate mixture of RNA molecules. Preferably these contain high replicating flank sequences such as those outlined above.

The kit may additionally comprise agents for breaking the emulsion and purification of amplified product.

### EXAMPLES

The following procedure describes the selection of RNA aptamers binding to streptavidin.

### RNA Library generation

As phage Qß replicase has a yet not well understood bias for replicatable elements, an existing sequence was used as a template for the design of a random library.

Central variable region of our libraries with flanking RQ fragments, T7 promoter for transcription (153nt with Bank40):

| | |
|---|---|
| (SEQ ID NO. 3) | TAATACGACTCACTATAGGGGTTCCAACCGGAAGTTGACACCGTACC TGCTCTNNNNNNNNNNNNNNNNNNNNNNNNNNNNNNNNNNNNNNNNA AGCACGCCAGGACTATCCGGTTTCGGACCTCCAGTGCGTGTTACCG CACTGTTAGCCC |

The resulting RNA transcript (136 nt with Bank40):

| | |
|---|---|
| (SEQ ID NO. 4) | GGGGUUCCAACCGGAAGUUGACACCGUACCUGCUCUNNNNNNNNNNN NNNNNNNNNNNNNNNNNNNNNNNNNNNNNAAGCACGCCAGGACUAU CCGGUUUCGGACCUCCAGUGCGUGUUACCGCACUGUUAGCCC |

Following oligonucleotides are needed for amplification of the library or reverse transcription before sequencing:

| | |
|---|---|
| TQPro5, 53nt (SEQ ID NO. 5) | TAATACGACTCACTATAGGGGTTCCAACCGGAAGTTGACACCGTACC TGCTCT |
| QPro3, 60nt (SEQ ID NO. 6) | GGGCTAACAGTGCGGTAACACGCACTGGAGGTCCGAAACCGGATAGT CCCTGGCGTGCTT |
| R3, 19nt (SEQ ID No. | GGGCTAACAGTGCGGTAAC |

### Affinity selection

The selection was performed on standard streptavidin magnetic beads obtained from Invitrogen.

The PCR is performed with Bank40 (40 variable nucleotides) as template with TQPro5 and QPro3 primers (2-3 cycles @60°C annealing temp). The resulting double-stranded DNA product is purified with a standard PCR cleanup kit. The template is transcribed with T7 RNA polymerase (Promega kit) and template DNA is digested by DNase 1.

The resulting RNA is purified with sbeadex magnetic beads obtained from AGOWA GmbH, Berlin, Germany and quantified with Nanodrop. The RNA Qbank40 is incubated with 50µl resuspended and pre-equilibrated streptavidin beads in binding buffer. The beads are washed with binding buffer. The retained RNA is eluted with 50µl Millipore water for 5 min at 70°C and beads are removed quickly. 24µl of the eluted sample is emulsified; the rest is kept at -20°C as a backup. An aliquot of 2µl may be used as an open control for Qβ amplification. This amplification process is described in more detail below. The selection is repeated additional 2 times until an RNA enrichment is observed. Each selection round is performed with increasing washing steps (1ml extra per selection round). After the selection, the enriched RNA is reverse transcribed for sequencing as described below. Amplification of RNA by Qbeta replicase The mixtures for amplification were prepared on ice and the enzyme was added shortly before the emulsion solutions to avoid premature amplification. The resulting mixture was vortexed for five minutes in a cold room before the replication was started at room temperature.

| Aqueous phase |
|---|
| 24µl) sample |
| 5µl 10xNEB buffer 4 |
| 20µl NTP (2.5mM each) |
| 1µl Qβ replicase (1u) |

| Oil surfactant mixture |
|---|
| 7% ABIL WE 09 |
| 20% mineral oil |
| 73% Tegosoft DEC |

For the amplification reaction add 50µl of aqueous phase to 300µl of oil surfactant mixture into a reaction tube. Each of these components needs to be prechilled on ice. The emulsification is performed in a cold room on a vortexer for 5min set at maximum speed. The amplification mixture is then incubated at 25°C for at least 4 hours to accumulate newly amplified RNA in the microdroplets. The emulsion is broken with 1.6ml butanol or isobutanol and the now homogeneous phase containing RNA with is purified with sbeadex magnetic beads. The results are checked on gel electrophoresis and quantified by nanodrop spectrometer.

### Reverse transcription from Qbank40 RNA

Generally, reverse transcription can be performed with any available reverse transcriptase. As the amount of template RNA was sufficient, the less efficient activity of Taq DNA polymerase was exploited: Perform RT-PCR with Taq DNA polymerase and Pro5+R3 primers according to Grabko et al. FEBS Letters 1996.

### Sequencing

The obtained PCR fragments from an unselected conventionally replicated RNA library and from the selection were batch sequenced by Sanger sequencing technology. The resulting sequences revealed that the conventional RNA consisted of a single sequence, whereas the streptavidin selection in emulsion displayed a reduced variability in comparison to the parental library. A subsequent high throughput sequencing experiment using Roche 454 technology revealed a strong bias for certain variants:
5633 reads total
1719 (30%) AGCCTTAGAGCCTGTGGTGTCCCGTTTAGCCTGGAGCGC (SEQ ID NO.8)
888 (15%) ATCTAATAGGGCATCCGTACGTTGTTCTTGACGATGCTTC (SEQ ID NO. 9)
328 ( 5%) AGCTGTCTGCAAGAGCACAACGGGGATGGCATGTTTGT (SEQ ID NO. 10)
185 ( 3%) GGTACATGTCTCTACTCACGTTAGTCATCTAGCTGCCATA (SEQ ID NO. 11)
170 ( 3%) ACAGCATGTGCGGTATCTCCACACCCAGTCCGTGCTTTAG (SEQ ID NO. 12)
163 ( 2%) TGCAAAAAGAACAGAGACCGTCAACTCTTTGTCGTGCTAG (SEQ ID NO. 13)
115 ( 2%) ATGAGCGCGATAAATAGTGTCACTCTCAGCCCGACTGCCT (SEQ ID NO. 14)
90 ( 1%) TGCAAAGGAGTGATACGGTGGTTCGGTGCTGGCTGTAAT (SEQ ID NO. 15)
53 ( 0%) GGAAGACAGTAATCTACCAACAGGCGGCAGACGTGCAAGT (SEQ ID NO. 16)
52 ( 0%) TGCAAAAGAACAGAGACCGTCAACTCTTTGTCGTGCTAG (SEQ ID NO. 17)
51 ( 0%) GTGACAATCAGGGCTGGTAGCGGGGACCCGACGTGTTGAT (SEQ ID NO. 18)
49 ( 0%) GATAAAGTGCAGGGCTAGTGCCTCTTCTGCGCGCTAATCT (SEQ ID NO. 19)
48 ( 0%) TTGAGGAGCAAGGGAGTGTAAAAAGACCCTCCCGCGCGTT (SEQ ID NO. 20)
27 ( 0%) TAATGAGGAGGCCGCCAACAGCCGTCCTGCATGCTATTAA (SEQ ID NO. 21)
26 ( 0%) CTGGGCAGGAGGGAAATCAACTGGACCTGGGTCGCTTTAT (SEQ ID NO. 22)
25 ( 0%) CGAAGTAGTATCGACGAGCGGTGTGGCTTGACGTTGCTAG (SEQ ID NO. 23)
24 ( 0%) ATGAGCGCGATAAATAGTGTCACTCTCAGCCTGACTGCCT (SEQ ID NO. 24)
20 ( 0%) ATGCCTATCATGGGC (SEQ ID NO. 25)
19 ( 0%) GTTAATGAAGATTACTGAGCAAGTGCGTGGGCCTGCCTAT (SEQ ID NO. 26)

### Binding assay

Three of the most prevalent clones were transcribed and tested in a fluorescent aptamer assay. All three tested clones displayed a biotin-dependent binding to streptavidin. The binding extent also positively correlated with the distribution of the clones in the final selection round.

## Claims

1. Method for isolating an RNA ligand from a candidate mixture of RNA molecules,
(i) each comprising a segment of least eight contiguous randomized nucleotides, being nucleotides of randomized type,
(ii) said RNA ligand being capable of binding a given target, the method comprising the steps of,
(iii) contacting the candidate mixture with one or more targets,
(iv) partitioning the unbound RNA molecules from the bound RNA ligands,
(v) amplifying the RNA ligands with an RNA dependant RNA polymerase in an amplification reaction fluid,
(vi) repeating steps (i) to (v) at least 2 times.

2. Method according to claim 1, wherein the amplification reaction fluid comprises micelles.

3. Method according to claim 1 or 2, wherein the micelles may comprise one or more of the following substances, an oil, a mineral oil, an ionic surfactant, a non-ionic surfactant, an organic solvent.

4. Method according to claim 3, wherein the surfactant is selected from the group of, an anionic surfactant based on sulfate, sulfonate or carboxylate anions, perfluorooctanoate (PFOA or PFO), perfluorooctanesulfonate (PFOS), ammonium lauryl sulfate, other alkyl sulfate salts, sodium laureth sulphate, alkyl benzene sulfonate, soaps, or fatty acid salts, cationic surfactants (based on quaternary ammonium cations), cetyl trimethylammonium bromide (CTAB) a.k.a. hexadecyl trimethyl ammonium bromide, alkyltrimethylammonium salts, cetylpyridinium chloride (CPC), polyethoxylated tallow amine (POEA), benzalkonium chloride (BAC), benzethonium chloride (BZT), zwitterionic (amphoteric), dodecyl betaine, cocamidopropyl betaine, coco ampho glycinate, nonionic surfactants, alkyl poly(ethylene oxide), alkylphenol poly(ethylene oxide), copolymers of poly(ethylene oxide) and poly(propylene oxide), alkyl polyglucosides, octyl glucoside, decyl maltoside, fatty alcohols, cetyl alcohol, oleyl alcohol, cocamide MEA, cocamide DEA, polysorbates, Tween 20, Tween 80 and dodecyl dimethylamine oxide.

5. Method according to claims 1 to 4, wherein the RNA dependant RNA polymerase in the amplification reaction fluid is Qß replicase.

6. Method according to claims 1 to 5, wherein the RNA molecules comprise a high replicating flank sequence.

7. Method according to claims 1 to 6, wherein the high replicating flank sequences are SEQ ID NO. 1 and 2.

8. Method according to claims 1 to 7, wherein one or more of the RNA ligands is sequenced directly without prior cloning.

9. Kit for isolating an RNA ligand from a candidate mixture of RNA molecules comprising a substance for generating micelles and an RNA dependant RNA replicating enzyme.

10. Kit according to claim 9, wherein the RNA replicating enzyme is Qß replicase.

11. Kit according to claim 9 or 10, wherein it additionally comprises a candidate mixture of RNA molecules.
